# EUROPEAN PATENT SPECIFICATION

(11) **EP 0 469 348 B1**
(45) Date of publication and mention of the grant of the patent: **12.03.1997**
(21) Application number: 91111413.0
(22) Date of filing: 09.07.1991
(51) Int. Cl.: C12N 15/51, C12Q 1/70

(54) **cDNA sequence and detection of hepatits C virus**
cDNS-Sequenz und Detektion des Hepatitis C-Virus
Séquence cADN et détection du virus de l'hépatite C

(30) Priority: 11.07.1990 JP 183512/90; 18.12.1990 JP 412177/90; 30.01.1991 JP 29364/91; 14.02.1991 JP 42855/91
(43) Date of publication of application: 05.02.1992
(73) Proprietor: SHIONOGI SEIYAKU KABUSHIKI KAISHA trading under the name of SHIONOGI & CO. LTD., Osaka 541 (JP)
(72) Inventor: Kamada, Takenobu, Osaka University, Fukushima-ku, Osaka-shi, Osaka (JP); Hayashi, Norio, Osaka University, Fukushima-ku, Osaka-shi, Osaka (JP); Mita, Eiji, Osaka University, Fukushima-ku, Osaka-shi, Osaka (JP); Ueda, Keiji, Osaka University, Fukushima-ku, Osaka-shi, Osaka (JP)
(74) Representative: VOSSIUS & PARTNER

(56) References cited:
- EP-A- 0 318 216
- EP-A- 0 388 232
- EP-A- 0 398 748
- EP-A- 0 419 182
- NUCLEIC ACIDS RESEARCH, no. 24, 1989, Arlington, VA (US); Y. KUBO et al., pp.
- 10367-10372
- THE LANCET, vol. 335, no. 8680, 06 January 1990, London (GB); A.J. WEINER et al., pp. 1-3

## Description

The present invention relates to a DNA sequence comprising a cDNA sequence derived from hepatitis C virus, which is designated by SEQ ID NO: 1, SEQ ID NO: 5, or SEQ ID NO: 10. The DNA sequence of the present invention is useful for the design and synthesis of probes and primers to be used in the RNA diagnosis of hepatitis C virus. This DNA sequence is also useful for the production of peptides to be used in the immunodiagnosis of hepatitis C by genetic engineering. Further, the peptides produced by genetic engineering with the use of this DNA sequence are useful for the production of antibodies to be used in the immunodiagnosis of hepatitis C.

The present invention also provides a method for the detection of hepatitis C virus, which is based on the PCR technique characterized by using one primer composed of from 15 to 40 bases including at least 15 straight bases in the DNA sequence designated by SEQ ID NO: 2 and the other primer composed of from 15 to 40 bases including at least 15 straight bases in the DNA sequence designated by SEQ ID NO: 3; or by using one primer composed of from 15 to 40 bases including at least 15 straight bases in the DNA sequence designated by SEQ ID NO: 15 and the other primer composed of from 15 to 40 bases including at least 15 straight bases in the DNA sequence designated by SEQ ID NO: 16.

Hepatitis A is an acute infectious disease which is caused by RNA virus of 27 nm in diameter and exhibits a good prognosis. Hepatitis A virus is present in liver parenchymal cells and stellate cells, and is excreted into feces so that they spread by oral infection. Because the period that the virus appears in blood is quite short, no infection is observed by way of blood. As compared with the acute hepatitis caused by other hepatitis viruses, the onset of this hepatitis is frequently accompanied by an attack of fever up to 38°C or more. In the majority of cases, the function of the liver will return to the normal conditions in about 40 days, although it may exhibit the rare progression to fulminant hepatitis.

Hepatitis B is caused by infection of hepatitis B virus. This virus falls under the category of DNA virus and has a double structure that consists of an envelope containing an HBs antigen and a core containing an HBc antigen. Infection of hepatitis B includes transient infection and persistent infection. In the case of transient infection, after the latent period of 1 to 6 months from the infection of hepatitis B virus, an increase in the serum GOT and GPT, as well as jaundice, will appear together with hepatitic symptoms consisting mainly of gastrointestinal symptoms. In most cases, the function of the liver will return to the normal conditions within 3 months after the onset of this disease; however, there are some cases where it may become fulminant hepatitis and exhibit the rapid progression to death. In the case of persistent infection, primary infection of hepatitis B virus may change to the persistent infection, or symptomatic carriers may suddenly exhibit symptoms of hepatitis. In this case, acute hepatitis with typical symptoms is rarely observed, while the insidious approach of chronic hepatitis or liver cirrhosis is frequently observed.

Non-A, non-B hepatitis is that other than hepatitis A and hepatitis B among the post-transfusion hepatitis and sporadic acute hepatitis which is believed to be caused by a virus, and non-A, non-B hepatitis has been considered to be caused by unknown viruses. In Japan, post-transfusion hepatitis occurs in a probability of from 13% to 20%, and non-A, non-B hepatitis accounts for 90% or more of these cases. Non-A, non-B hepatitis has a stronger tendency to become chronic than hepatitis B. In many cases, the symptoms of this hepatitis are more anicteric and further lack subjective symptoms as compared with hepatitis A and hepatitis B (Menekigaku Yougo Jiten , Second edition, SAISHIN-IGAKU Publishing Co., Ltd.).

As described above, non-A, non-B hepatitis has been considered as the hepatitis which will be caused by unknown viruses. Recently, Houghton et al. (EPO Pub. No. 0 318 216) have succeeded in isolating a part of cDNA derived from the genome of non-A, non-B hepatitis virus by screening the expression products from a cDNA library which was obtained from the infected tissue and sera of patients with non-A, non-B hepatitis, and this hepatitis virus was named hepatitis C virus (HCV). Further, Kubo et al. (Nucleic Acids Research, 17 (1989) 10367-10372) has succeeded in isolating a part of cDNA derived from the genome of HCV different from the above-mentioned HCV.

As described above, it has been suggested that HCV is not a single virus and there are many subtypes of HCV. It has also been suggested that there are different subtypes of HCV depending upon the areas. Accordingly, for the purpose of attaining the accurate diagnosis, prevention of transmission, and treatment of hepatitis C, as well as its elucidation, HCV of an additional subtype should be isolated. A method for detecting HCV of such a subtype is also required.

The objective of the present invention is to provide a cDNA derived from an HCV of the subtype which is different from that of the known HCV. The DNA sequence of the present invention comprises the HCV cDNA sequence designated by SEQ ID NO: 1, SEQ ID NO: 5, or SEQ ID NO: 10 and more particularly, it consists of the DNA sequence designated by SEQ ID NO: 1, SEQ ID NO: 5, or SEQ ID NO: 10. The DNA sequence of the present invention is useful for the design and synthesis of probes and primers to be used in the RNA diagnosis of HCV, and also useful for the production of peptides to be used in the immunodiagnosis of hepatitis C by genetic engineering. Further, the peptides produced by genetic engineering with the use of this DNA are also useful for the production of antibodies to be used in the immunodiagnosis of hepatitis C. Because the DNA sequence is different from the known HCV cDNA, the DNA sequence of the present invention is particularly useful for the diagnosis of hepatitis C which cannot be detected with reference to the known HCV cDNA sequence. In addition, from the comparison between the DNA sequence of the present invention and the known cDNA, if primers and probes with a higher homology to the both are prepared, it becomes possible to conduct the extensive RNA diagnosis of hepatitis C.

The present invention further provides a method for the detection of hepatitis C viruses which is based on the PCR technique characterized by using a primer composed of from 15 to 40 bases including at least 15 straight bases in the DNA sequence designated by SEQ ID NO: 2 and a primer composed of from 15 to 40 bases including at least 15 straight bases in the DNA sequence designated by SEQ ID NO: 3; or by using a primer composed of from 15 to 40 bases including at least 15 straight bases in the DNA sequence designated by SEQ ID NO: 15 and a primer composed of from 15 to 40 bases including at least 15 straight bases in the DNA sequence designated by SEQ ID NO: 16.

The HCV cDNA of the present invention is isolated according to the following process.
(1) RNA extraction
   The pellets obtained from sera of HCV-antibody positive patients by ultracentrifugation or normal portions of the removed liver of a patient with liver cancer are homogenized in an appropriate buffer such as GIT buffer, followed by ultracentrifugation, and the resulting pellets are dissolved in an appropriate buffer such as TE buffer, after which RNA is extracted by phenol extraction and ethanol precipitation.
(2) Reverse transcription
   The RNA obtained in Item (1) and an anti-sense primer synthesized on the basis of the known HCV cDNA sequence are allowed to react with a commercially available reverse transcriptase, thereby attaining reverse transcription.
(3) Polymerase chain reaction
   To the reaction solution obtained in Item (2), a sense primer synthesized on the basis of the known HCV cDNA sequence and Taq DNA polymerase are added, and the polymerase chain reaction is conducted according to a conventional procedure.
(4) Second PCR
   As occasion demands, PCR product obtained in (3) is again subjected to PCR using sense/anti-sense primers different from or same as those used in (3).
(5) Electrophoresis
   The first or second PCR product is subjected to electrophoresis by use of a polyacrylamide gel or the like, and a band having a chain length which is deduced from the primer used is cut out, thereby obtaining DNA having the desired chain length.
(5) Cloning
   The DNA obtained in Item (5), preferably phosphorylated at 5' ends, is inserted into a commercially available plasmid containing an appropriate selection marker, and the resulting plasmid is introduced into a suitable host such as Escherichia coli. The chain length of a plasmid DNA insert contained in transformants is determined, and clones containing the DNA insert of the desired chain length are identified as positive.
(7) Sequencing
   The base sequence of the DNA insert in the positive clone is determined by the conventional dideoxy chain termination method.

The base sequence of the cDNA of the present invention is designated by SEQ ID NO: 1, SEQ ID NO: 5, or SEQ ID NO: 10. From the comparison between the cDNA sequence of the present invention (SEQ ID NO: 1) and the HCV cDNA sequence previously reported (J1: Nucleic Acids Research , 17 (1989) 10367-10372, and PT: EPO Pub. No. 0 318 216), it was found that M16 (SEQ ID NO: 1) of the present invention showed about 96% and about 81% homology to J1 and PT, respectively, from which the present inventors inferred that M16 would be a part of a novel HCV cDNA. Further, the base sequences of SEQ ID NO: 5 and SEQ ID NO: 10 are identified as novel.

The present invention further provides a method for the detection of hepatitis C viruses which is based on the PCR technique characterized by using a primer composed of from 15 to 40 bases including at least 15 straight bases in the DNA sequence designated by SEQ ID NO: 2 and a primer composed of from 15 to 40 bases including at least 15 straight bases in the DNA sequence designated by SEQ ID NO: 3; or by using a primer composed of from 15 to 40 bases including at least 15 straight bases in the DNA sequence designated by SEQ ID NO: 15 and a primer composed of from 15 to 40 bases including at least 15 straight bases in the DNA sequence designated by SEQ ID NO: 16.

The DNA sequence designated by SEQ ID NO: 2 is an anti-sense sequence corresponding to bases 604-623 of the DNA sequence designated by SEQ ID NO: 1, while the DNA sequence designated by SEQ ID NO: 3 is a sense sequence corresponding to bases 1-20 of the DNA sequence designated by SEQ ID NO: 1.

The DNA sequence designated by SEQ ID NO: 15 is an anti-sense sequence corresponding to bases 309-328 of the DNA sequence designated by SEQ ID NO: 10, while the DNA sequence designated by SEQ ID NO: 16 is a sense sequence corresponding to bases 9-28 of the DNA sequence designated by SEQ ID NO: 10.

The primers used in the method for the detection of hepatitis C virus according to the present invention are not limited to the DNA sequences designated by SEQ ID NO: 2 and SEQ ID NO: 3 or SEQ ID NO: 15 and SEQ ID NO: 16, and those composed of from 15 to 45 bases including at least 15 continuous bases of these DNA sequences can also be used. A chain length of from 15 to 40 bases is conventional for primers to be used in the PCR technique. It is also possible to use a combination of primers complementary to the DNA sequences described by SEQ ID NO: 2 and SEQ ID NO: 3 or SEQ ID NO: 15 and SEQ ID NO: 16. The design and preparation of such primers can be conveniently achieved on the basis of the DNA sequence described by SEQ ID NO: 1 or SEQ ID NO: 10 so that the DNA sequence described by SEQ ID NO: 1 or SEQ ID NO: 10 can be specifically detected by the PCR technique.

As the PCR technique used in the present invention, preferred is a reverse transcription-polymerase chain reaction (RT-PCR) method in which reverse transcription is carried out prior to PCR, because hepatitis C virus falls under the category of RNA virus.

The detection of PCR products can be achieved by subjecting to electrophoresis by use of a gel and southern hybridization using a probe corresponding to SEQ ID NO: 1, e.g., a probe of the sequence described by SEQ ID NO: 4; or a probe corresponding to SEQ ID NO: 10, e.g., a probe of the sequence described by SEQ ID NO: 17 which corresponds to bases 83-106 shown in SEQ ID NO: 10. In place of the southern hybridization, PCR product can be detected by subjecting to double PCR in which first PCR product is again subjected to PCR, electrophoresing the product with the use of a gel containing ethidium bromide, and detecting under ultraviolet light.

The present invention will be further illustrated by reference to the following example; however, this example is not intended to restrict the scope of the present invention.

### EXAMPLE

### Example 1

### (1) RNA extraction

Sera from ten HCV-antibody positive patients at each volume of about 2 ml (the total volume being about 20 ml) were diluted 7 times with a TEN buffer (10 mM Tris-HCl (pH 7.5), 1 mM EDTA, 0.1 M NaCl), and then pelleted by ultracentrifugation at 36,000 rpm for 2 hours at 15°C with an HITACHI RPS40T2 rotor. The resulting pellets and about 1 g of normal portions of the removed liver of HCV-antibody positive patients with liver cancer were homogenized in 40 ml of GIT buffer (4.0 M guanidinium thiocyanate, 0.1 M Tris-HCl (pH 7.8), 1% β -mercaptoethanol, and 0.5% sodium N-lauroylsarcosinate) with a blender (manufactured by KINEMATIC Co.) at the maximum speed for about 30 seconds.

Next, 20 ml of the homogenized solution was slowly overlaid over 20 ml of 5.7 M CsCl-0.01 M EDTA (pH 8.0), and then subjected to ultracentrifugation with a HITACHI SRP-28SA rotor at 24,000 rpm for 24 hours at 15°C. Then, about 800 µg of the resulting pellet was dissolved in a diethylpyrocarbonate-treated TE buffer (10 mM Tris-HCl (pH 7.8) and 1 mM EDTA), and extracted with phenol/chloroform/isoamylalcohol (24:24:1), followed by ethanol precipitation.

The resulting precipitate was dissolved in a diethylpyrocarbonate-treated TE buffer; after the concentration of nucleic acid was determined, the solution obtained was adjusted to an RNA concentration being 1 µg per µl of TE buffer; and the adjusted solution was used for the subsequent reverse transcription.

### (2) Reverse transcription

First, 1 µl of RNA ( 1 µg/µl TE buffer) obtained in the above-mentioned step (1) was mixed with a reaction solution of the composition:

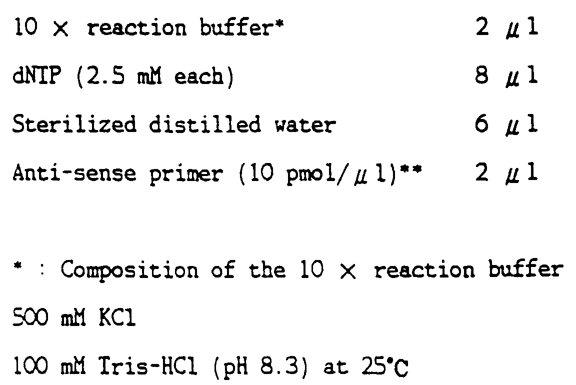

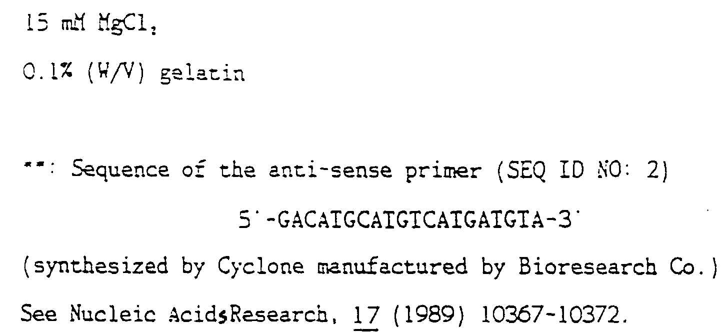

The above-mentioned reaction solution was preheated at 65°C for 5 minutes to release the secondary structure of RNA, after which the solution was allowed to stand at room temperature for 5 minutes to anneal the primer. To this solution, 1 µl of 200 units/µl M-MLV reverse transcriptase (Bethesda Research Laboratories) was added, and reverse transcription was conducted at 37°C for 60 minutes.

### (3) Polymerase chain reaction (PCR)

To 20 µl of the reaction solution obtained in the above-mentioned step (2), the following solution was added.

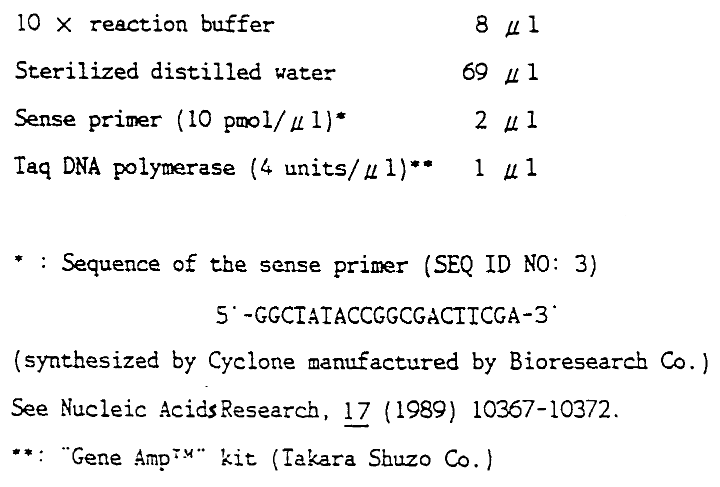

The PCR was conducted 30 cycles under the conditions of temperature, 94°C for 30 seconds, 55°C for 1 minute, and 72°C for 1 minute, followed by additional treatment at 72°C for 15 minutes.

### (4) Electrophoresis

First, 100 µl of the PCR product was extracted with 100 µl of phenol/chloroform/isoamylalcohol (24:24:1), followed by ethanol precipitation. Then, about 500 ng of the precipitate was suspended in 10 µl of TE buffer, to which bromophenol blue/xylenecyanol/sucrose (4 µl) was added, thereby obtaining a sample.

Separately, 2.8 ml of 30% acrylamide solution (29% acrylamide and 1% N, N'-methylene-bis-acrylamide), 2.4 ml of 10 × TBE buffer (890 mM Tris-boric acid and 20 mM EDTA (pH 8.0)), and 18.8 ml of sterilized distilled water were mixed together, to which a small amount of ammonium persulfate and 20 µl of N, N, N', N'-tetramethylethylenediamine were added, thereby obtaining 3.5% polyacrylamide gel.

Using this gel, the above-mentioned sample was subjected to electrophoresis, after which double-stranded DNA was stained with ethidium bromide and observed under ultraviolet light. At the same time, as size markers, AluI-digested pBR322 (containing 908 bp, 659 bp, 656 bp, or 521 bp fragments) was subjected to electrophoresis. The desired band near the position corresponding to 623 bp which was deduced from the primer used (single band) was cut out and dissolved in 20 µl of TE buffer.

### (5) Cloning

First, 1 µl of 2 µg/µl pBluescript II SK(⁺) (manufactured by Stratagene Co. ) was mixed with 2 µl of a reaction mixture (containing 100 mM Tris-HCl, 70 mM MgCl₂, 200 mM KCl, 70 mM 2-mercaptoethanol, and 1 mg/ml bovine serum albumin), 16 µl of sterilized distilled water, and 1 µl. of SmaI (14 unit/µl, manufactured by Toyobo Co.), and the mixture was incubated at 30°C for 1 hour to digest with SmaI, followed by phenol extraction and ethanol precipitation, after which the resulting precipitate was suspended in a TE buffer (final concentration, 0.1 µg/µl).

Then, 1 µl of the SmaI-digested pBluescript II SK(⁺) solution was mixed with 6 µl of the DNA solution obtained in the above-mentioned step (4), 2 µl of 5 × ligation buffer (250 mM Tris-HCl (pH 7.8), 50 mM MgCl₂, 50 mM dithiothreitol, 2.5 mM ATP, and 500 µg/ml bovine serum albumin), and 1 µl of T₄DNA ligase (5 units/µl), and the mixture was incubated at 16°C for 20 hours.

Ca⁺⁺-treated E. coli XL-1-B (100 µl) was mixed with the above-mentioned reaction mixture (1 µl), and the mixture was allowed to stand on ice at 0°C for 30 minutes, followed by transformation on an L plate containing 40 µg/ml X-gal, 40 µg/ml IPTG, and 50 µg/ml ampicillin.

The transformed white colonies were incubated overnight in 5 ml of L medium. A 1.5 ml portion of this culture was centrifuged at 7000 rpm for 40 seconds. The resulting pellets was suspended in 100 µl of a STET buffer (8% sucrose, 5% Triton X-100, 50 mM EDTA, and 50 mM Tris-HCl (pH 8.0)). To this suspension, 10 µl of 10 µg/µl lysozyme was added, and the mixture was boiled for 40 seconds. The mixture was then extracted with an equal volume of phenol/chloroform/isoamylalcohol (24:24:1), after which an equal volume of isopropanol was added to the resulting supernatant and the mixture was allowed to stand at -20°C for 30 minutes. After centrifugation (15,000 rpm, 4°C, 10 minutes), the resulting pellets were suspended in 100 µl of a TE buffer. To 16 µl of this suspension, 2 µl of 10 × High buffer (500 mM Tris-HCl (pH 7.5), 1000 mM NaCl, 100 mM MgCl₂, and 10 mM dithiothreitol), 1 µl or BamHI (12 units/µl), and 1 µl of EcoRI (12 units/µl) were added, and the mixture was incubated at 37°C for 30 minutes, resulting in a double digestion with BamHI and EcoRI. To 20 µl of this reaction mixture, bromophenol blue/xylenecyanol/sucrose (5 µl) was added, and the mixture was subjected to electrophoresis using a 1.2% agarose gel. As a size marker, AluI-digested pBR322 was used. After staining with ethidium bromide, 2 positive clones were obtained from a band appearing around the desired size of 623 bp by observation under ultraviolet light.

### (6) Sequencing

The base sequence of a DNA insert of M16, which is one of the positive clones obtained in the above-mentioned step (5), was determined by the dideoxy chain termination method. The base sequence determined is shown in the sequence listing, SEQ ID NO. 1. From the comparison between the DNA sequence of the present invention and the previously reported HCV cDNA sequence (J1: Nucleic Acids Research, 17 (1989) 10367-10372, and PT: EPO Pub. No. 0 318 216), M16 obtained in the present invention showed about 96% and about 81% homology to J1 and PT, respectively, and it was presumed to be a part of a novel HCV cDNA.

### Example 2

### (1) RNA extraction

About 1 g of normal portions of the removed liver of a patient with liver cancer was homogenized in 40 ml of GIT buffer (4.0 M guanidinium thiocyanate, 0.1 M Tris-HCl (pH 7.8), and 1% β-mercaptoethanol) with a blender (manufactured by KINEMATIC Co.) at the maximum speed for about 30 seconds. To this homogenized solution is added 10% sodium N-lauroylsarcosinate solution to give its final concentration of 0.5 %.

Next, 20 ml of the solution was slowly overlaid over 20 ml of 5.7 M CsCl-0.01 M EDTA (pH 8.0), and then subjected to ultracentrifugation with a HITACHI SRP-28SA rotor at 24,000 rpm for 24 hours at 15 °C. Then, the resulting pellet was dissolved in a diethylpyrocarbonate-treated TE buffer (100 mM Tris-HCl (pH 7.8) and 1 mM EDTA), and extracted with phenol/chloroform/isoamylalcohol (24:24:1), followed by ethanol precipitation.

The resulting precipitate was dissolved in 300 µl of a diethylpyrocarbonate-treated TE buffer. Its concentration of nucleic acid was determined. The RNA concentration was 2.8 µg/µl TE buffer.

### (2) Reverse transcription

First, 1 µl of RNA ( 2.8 µg/µl) obtained in the above-mentioned step (1) was mixed with a reaction solution of the composition:

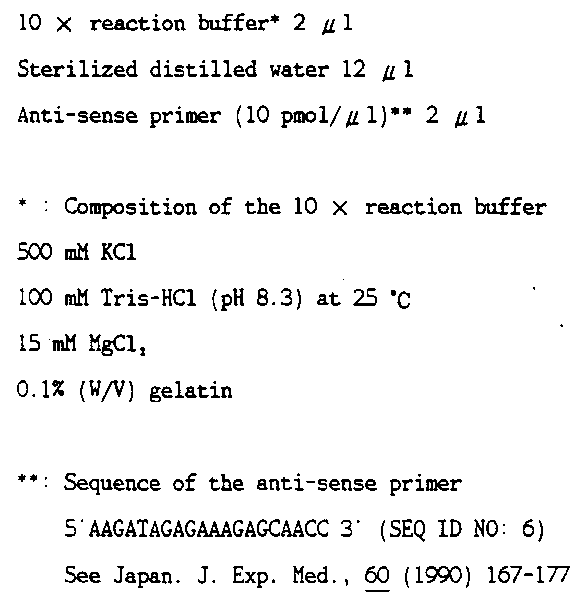

The above-mentioned reaction solution was preheated at 65 °C for 5 minutes to release the secondary structure of RNA, after which the solution was allowed to stand at room temperature for 2 minutes to anneal the primer. To this solution, the following solution was added, and reverse transcription was conducted at 42°C for 60 minutes.

| | |
|---|---|
| dNTP (10 mM each) | 2 µl |
| RNase inhibitor (40 units/µl)* | 0.5 µl |
| M-MLV reverse transcriptase (200 units/µl)** | 0.5 µl |

| | |
|---|---|
| *: Promega Co. | |
| **: Bethesda Research Laboratories | |

### (3) Polymerase chain reaction (PCR)

To 20 µl of the reaction solution obtained in the above-mentioned step (2), the following solution was added.

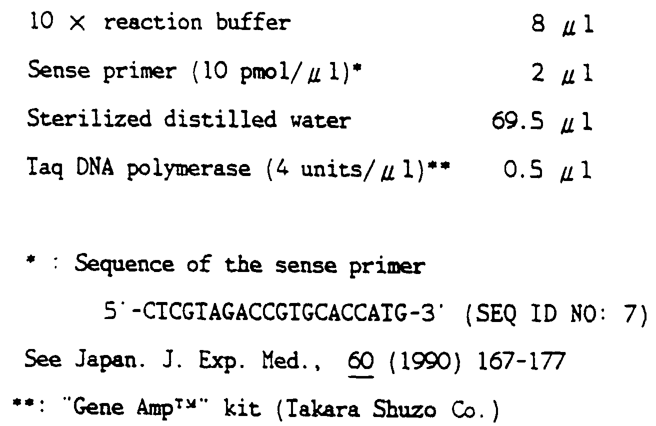

The PCR was conducted 30 cycles with 1 cycle being
denaturation: 94 °C for 30 seconds,
annealing: 55 °C for 1 minute, and
extension: 72 °C for 1 minute,
followed by additional treatment at 72 °C for 15 minutes.

### (4) Second PCR

To 5 µl of the reaction solution obtained in the above-mentioned step (3), the following solution was added.

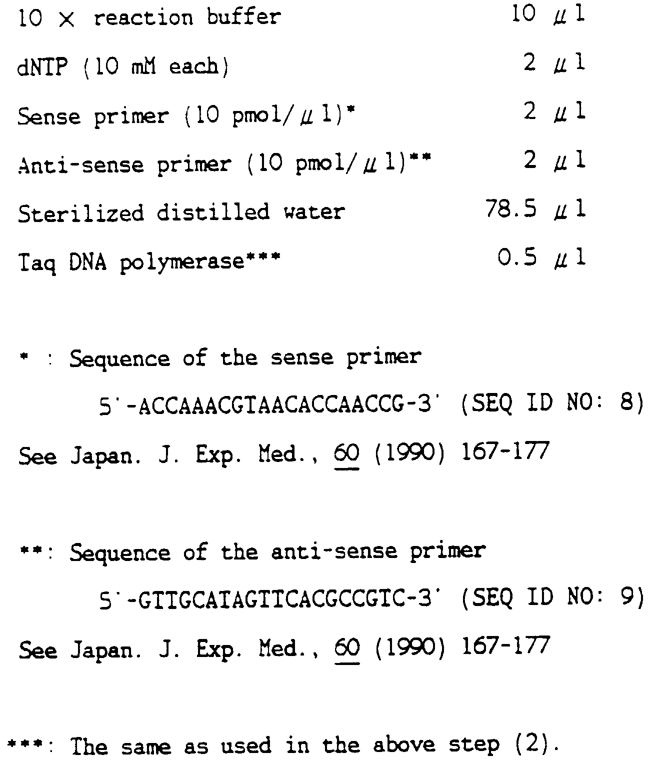

The PCR was conducted 30 cycles with 1 cycle being
denaturation: 94 °C for 30 seconds,
annealing: 55 °C for 1 minute, and
extension: 72 °C for 1 minute,
followed by additional treatment at 72 °C for 15 minutes.

### (5) Electrophoresis

To 60 µl of the PCR product of the above step (4) was added bromophenol blue/xylenecyanol/sucrose (20 µl), which was electrophoresed with the use of an agarose gel (Sea Plaque, FMC Co.) and stained with ethidium bromide. Double-stranded DNA was observed under ultraviolet light. At the same time, as a size marker, AluI-digested pBR322 (containing 908 bp, 659 bp, 656 bp, or 521 bp fragments) was subjected to electrophoresis. With reference to these bands, a single band of the desired length was cut out, DNA in which was recovered according to the method of Sambrook et al. (Molecular Cloning, Cold Spring Harbour Laboratory (CSHL) Press).

### (6) Cloning

### Kination

A solution (10 µl) of 0.2 µg/µl DNA obtained in the above step (5) in TE buffer was mixed with the following solutions.

| | |
|---|---|
| 5 × Kination buffer* | 4 µl |
| ATP (50 pmol/µl) | 2 µl |
| Sterilized distilled water | 2 µl |
| T₄ polynucleotide kinase (10 units/µl)** | 2 µl |

| | |
|---|---|
| *: Composition of 5×Kination buffer 250mM Tris-HCl (pH 9.5) 50mM MgCl₂ 25mM DTT 25% glycerol | |
| **: Bio Labs. | |

The mixture obtained above was allowed to react at 37 °C for 30 minutes, to which was added 2 µl of T₄ polynucleotide kinase. This was further allowed to react at 37 °C for 30 minutes to phosphorylate DNA at 5' ends.

The resultant was treated with phenol and precipitated with ethanol.

### Ligation

A solution (1 µl) of 0.05 µg/µl of the precipitate obtained by the above ethanol-precipitation in TE buffer was mixed with the following solutions.

| | |
|---|---|
| 5×ligation buffer* | 2 µl |
| CIAP-treated/SmaI-digested-pBluescript II SK(⁺)** | 1 µl |
| Sterilized distilled water | 5 µl |
| T₄ DNA ligase (5 units/µl) | 1 µl |

| | |
|---|---|
| *: Composition of 5×ligation buffer 250 mM Tris-HCl (pH 7.8) 50 mM MgCl₂ 50 mM dithiothreitol 2.5 mM ATP 500 µg/ml bovine serum albumin | |
| **: pBluescript II SK(⁺) (Stratagene) completely digested with SmaI, dephosphorylated with calf intestinal alkaline phosphatase according to the method of Sambrook et al. (Molecular Cloning, CSHL Press), and dissolved in TE buffer to give a concentration of 0.1 µg/µl. | |

This mixture was allowed to react at 16 °C for 24 hours.

### Transformation

Ca⁺⁺-treated E. coli XL-1-B (100 µl) was mixed with the above-mentioned reaction mixture (1 µl), and the mixture was allowed to stand on ice at 0 °C for 30 minutes, followed by transformation on an L plate containing 40 µg/ml X-gal, 40 µg/ml IPTG, and 50 µg/ml ampicillin.

The transformed white colonies were incubated overnight in 5 ml of L medium. A 1.5 ml-portion of this culture was centrifuged at 7000 rpm for 40 seconds. The resulting pellets was suspended in 100 µl of a STET buffer (8% sucrose, 5% Triton X-100, 50 mM EDTA and 50 mM Tris-HCl (pH 8.0)). To this suspension, 10 µl of 10 µg/µl lysozyme was added, and the mixture was boiled for 40 seconds. The mixture was then extracted with an equal volume of phenol/chloroform/isoamylalcohol (24:24:1), after which an equal volume of isopropanol was added to the resulting supernatant and the mixture was allowed to stand at -20 °C for 30 minutes. After centrifugation (15,000 rpm, 4 °C, 10 minutes), the resulting pellets were suspended in 100 µl of a TE buffer. To 16 µl of this suspension, 2 µl of 10 × High buffer (500 mM Tris-HCl (pH 7.5), 1000 mM NaCl, 100 mM MgCl₂, and 10 mM dithiothreitol), 1 µl of BamHI (12 units/µl), and 1 µl of EcoRI (12 units/µl) were added, and the mixture was incubated at 37 °C for 1 hour, resulting in a double digestion with BamHI and EcoRI.

To 20 µl of this reaction mixture, bromophenol blue/ xylenecyanol/ sucrose (5 µl) was added, and the mixture was subjected to electrophoresis using a 1.2% agarose gel. As a size marker, AluI-digested pBR322 was used. After staining with ethidium bromide, a band of the desired length was observed under ultraviolet light. Clones corresponding to the band were identified as positive.

### (7) Sequencing

The base sequence of a DNA insert of M20, which is one of the positive clones obtained in the above-mentioned step (6), was determined by the dideoxy chain termination method. The base sequence determined is shown in the sequence listing, SEQ ID NO: 5.

### Example 3

### (1) RNA extraction

About 1 g of normal portions of the removed liver of a patient with liver cancer was homogenized in 40 ml of GIT buffer (4.0 M guanidinium thiocyanate, 0.1 M Tris-HCl (pH 7.5), and 1% β-mercaptoethanol) with a blender (manufactured by KINEMATIC Co.) at the maximum speed for about 30 seconds. To this homogenized solution, 10 % sodium N-lauroylsarcosinate solution is added to give its concentration of 0.5 %.

Next, 20 ml of the solution was slowly overlaid over 20 ml of 5.7 M CsCl-0.01 M EDTA (pH 8.0), and then subjected to ultracentrifugation with a HITACHI SRP-28SA rotor at 24,000 rpm for 24 hours at 15 °C. The resulting pellet was dissolved in a diethylpyrocarbonate-treated TE buffer (100 mM Tris-HCl (pH 7.8) and 1 mM EDTA), and extracted with phenol/chloroform/isoamylalcohol (24:24:1), followed by ethanol precipitation.

The resulting precipitate was dissolved in 300 µl of a diethylpyrocarbonate-treated TE buffer. The concentration of nucleic acid was determined. The RNA concentration was 2.8 µg per µl TE buffer.

### (2) Reverse transcription

First, 1 µl of RNA (2.8 µg/µl) obtained in the above-mentioned step (1) was mixed with a reaction solution of the composition:

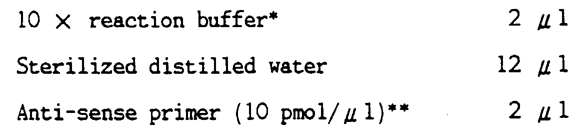

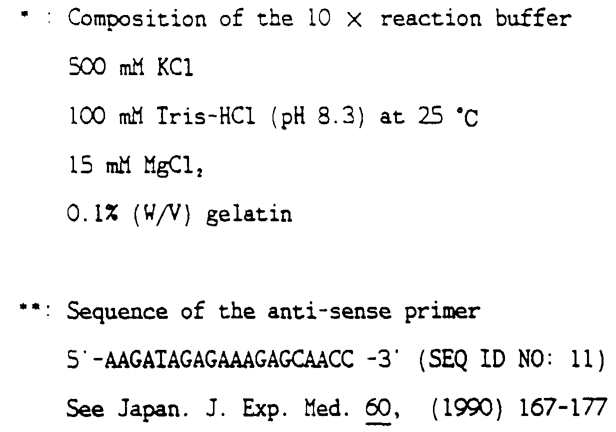

The above-mentioned reaction solution was preheated at 65 °C for 5 minutes to release the secondary structure of RNA, after which the solution was allowed to stand at room temperature for 2 hours to anneal the primer. To this solution the following solutions were added, and reverse transcription was conducted at 42 °C for 60 minutes.

| | |
|---|---|
| dNTP (10 mM each) | 2 µl |
| RNase inhibitor (40 units/µl)* | 0.5 µl |
| M-MLV reverse transcriptase (200 units/ µl)** | 0.5 µl |

| | |
|---|---|
| *: Promega Co. | |
| ** : Bethesda Research Laboratories | |

### (3) Polymerase chain reaction (PCR)

To 20 µl of the reaction solution obtained in the above-mentioned step (2), the following solutions were added.

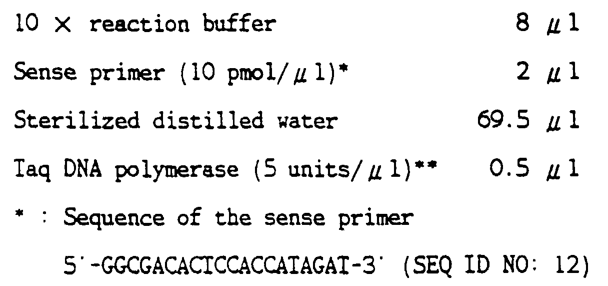

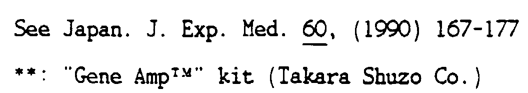

The PCR was conducted 30 cycles with one cycle being
Denaturation: 94 °C for 30 seconds,
Annealing: 55 °C for 1 minute, and
Extension: 72 °C for 1 minute,
followed by additional treatment at 72 °C for 15 minutes.

### (4) Second PCR

To 5 µl of the reaction solution obtained in the above-mentioned step (3), the following solutions were added.

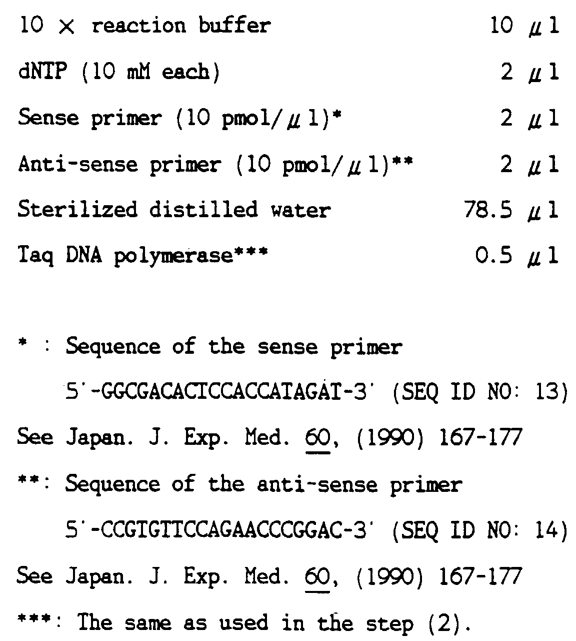

The PCR was conducted 30 cycles with one cycle being

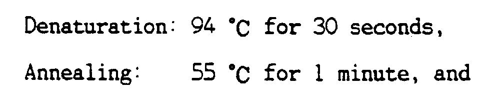

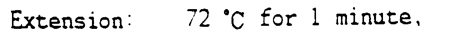

followed by additional treatment at 72 °C for 15 minutes.

### (5) Electrophoresis

To 60 µl of the PCR product obtained in the above step (4) was added 20 µl of bromophenol blue/xylenecyanol/sucrose. This was subjected to electrophoresis using an agarose gel (Sea Plaque, FMC Co.), after which double-stranded DNA was stained with ethidium bromide and observed under ultraviolet light. At the same time, as a size marker, AluI-digested pBR322 (containing 908 bp, 659 bp, 656 bp, or 521 bp fragments) was subjected to electrophoresis. With reference to these fragments, a single band of the desired length was cut out. The DNA in the band was recovered according to the method of Sambrook et al. (Molecular Cloning, Cold Spring Harbour Laboratory (CSHL) Press).

### (6) Cloning

### Kination

A solution (10 µl) of 0.2 µg/µl DNA obtained in the above step (5) in TE buffer was mixed with the following solutions.

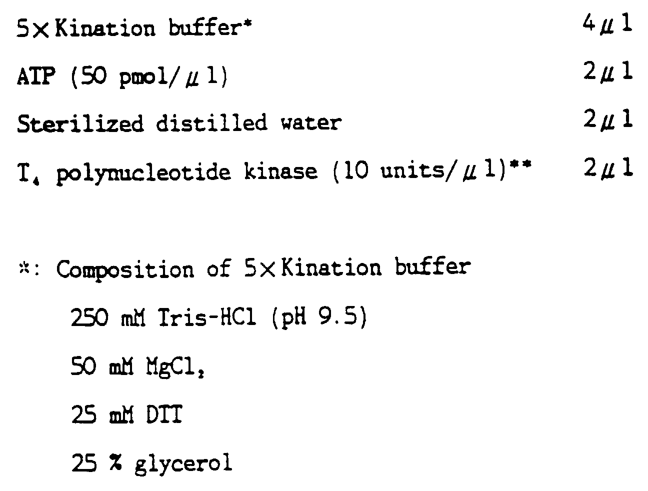

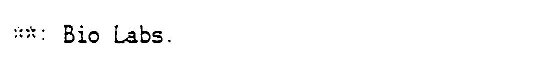

The mixture obtained above was allowed to react at 37 °C for 30 minutes, to which was added 2 µl of T₄ polynucleotide kinase. This was further allowed to react at 37 °C for 30 minutes to phosphorylate DNA at 5' ends.

The resultant was treated with phenol and precipitated with ethanol.

### Ligation

A solution (1 µl) of 0.05 µg/µl of the precipitate obtained by the above ethanol-precipitation in TE buffer was mixed with the following solutions.

| | |
|---|---|
| 5×ligation buffer* | 2 µl |
| CIAP-treated/SmaI-digested-pBluescript II SK(⁺)** | 1 µl |
| Sterilized distilled water | 5 µl |
| T₄ DNA ligase (5 units/µl) | 1 µl |

| | |
|---|---|
| *: Composition of 5×ligation buffer 250 mM Tris-HCl (pH 7.8) 50 mM MgCl₂ 50 mM dithiothreitol 2.5 mM ATP 500 µg/ml bovine serum albumin | |
| **: pBluescript II SK(⁺) (Stratagene) completely digested with SmaI, dephosphorylated with calf intestinal alkaline phosphatase according to the method of Sambrook et al. (Molecular Cloning, CSHL Press), and dissolved in TE buffer to give a concentration of 0.1 µg/µl. | |

This mixture was allowed to react at 16 °C for 24 hours.

### Transformation

Ca⁺⁺-treated E. coli XL-1-B (100 µl) was mixed with the above-mentioned ligating reaction mixture (1 µl), and the mixture was allowed to stand on ice at 0 °C for 30 minutes, followed by transformation on an L plate containing 40 µg/ml X-gal, 40 µg/ml IPTG, and 50 µg/ml ampicillin.

The transformed white colonies were incubated overnight in 5 ml of an L medium. A 1.5 ml portion of this culture was centrifuged at 7000 rpm for 40 seconds. The resulting pellet was suspended in 100 µl of an STET buffer (8% sucrose, 5% Triton X-100, 50 mM EDTA, and 50 mM Tris-HCl (pH 8.0)). To this suspension, 10 µl of 10 µg/µl lysozyme was added, and the mixture was boiled for 40 seconds. The mixture was then extracted with an equal volume of phenol/chloroform/isoamylalcohol (24:24:1), after which an equal volume of isopropanol was added to the resulting supernatant and the mixture was allowed to stand at -20 °C for 30 minutes. After centrifugation (15,000 rpm, 4 °C, 10 minutes), the resulting pellet was suspended in 100 µl of a TE buffer. To 16 µl of this suspension, 2 µl of 10 × High buffer (500 mM Tris-HCl (pH 7.5), 1000 mM NaCl, 100 mM MgCl₂, and 10 mM dithiothreitol), 1 µl of BamHI (12 units/µl), and 1 µl of EcoRI (12 units/µl) were added, and the mixture was incubated at 37 °C for 1 hour, resulting in a double digestion with BamHI and EcoRI.

To 20 µl of this reaction mixture, bromophenol blue/ xylenecyanol/ sucrose (5 µl) was added, and the mixture was subjected to electrophoresis using a 1.2% agarose gel. As a size marker, AluI-digested pBR322 was used. After staining with ethidium bromide, a band around the desired length was observed under ultraviolet light. Clones corresponding to the band were identified as positive.

### (7) Sequencing

The base sequence of a DNA insert of M642, which is one of the positive clones obtained in the above-mentioned step (6), was determined by the dideoxy chain termination method. The base sequence determined is shown in SEQ ID NO: 10.

### Example 4

### Detection of Hepatitis C Virus According to RT-PCR Method

An experiment of the following steps was carried out on 115 cases of non-A, non-B-type chronic liver disease and 10 cases of B-type chronic liver disease.

### (1) RNA extraction

To 200 µl of serum of each patient mentioned above was added 100 µl of 21% PEG 6000 - 1.5M NaCl. This mixture was mixed by a tube mixer, vortex, centrifuged lightly, and allowed to stand in ice for 1 - 2 hours. After centrifugation with 15,000 rpm at 4 °C for 20 minutes (Tomy, Angle rotor) to remove the resulting supernatant, to the obtained precipitate was added 400 µl of GTC buffer (4M guanidinium thiocyanate, 0.1 M Tris-HCl (pH 7.5), 1% β- mercaptoethanol, and 0.5 % sarcosyl). After dissolving the precipitate by pipetting, this mixture was briefly centrifuged. To this was added 400 µl of phenol/ chloroform/ isoamylalcohol (24:24:1). This mixture was mixed by vortex and centrifuged with 12,000 rpm at room temperature for 20 minutes (Tomy, Swing rotor) to recover the resulting supernatant (water layer).

To the supernatant was further added an equal volume of phenol/ chloroform/ isoamylalcohol (24:24:1). This mixture was mixed by vortex and centrifuged with 12,000 rpm at room temperature for 20 minutes (Tomy, Swing rotor) to recover 300 µl of the resulting supernatant (water layer). To the supernatant was added 1 µl of tRNA (10 µg/µl), 1/10 volume (30 µl) of 3 M sodium acetate, and 2.5 volumes (750 µl) of cold ethanol. This mixture was allowed to stand at -80 °C for 1 hour or more and centrifuged with 15,000 rpm at 4 °C for 15 minutes (Tomy, Angle rotor) to obtain a precipitate. To the precipitate was added 200 µl of 70 % ethanol. This mixture was briefly mixed by vortex and centrifuged with 15,000 rpm at 4 °C for 10 minutes (Tomy, Angle rotor) to obtain a precipitate. The precipitate was dried in a vacuum in a desiccator, dissolved in RT solution I*, heated at 65 °C for 10 minutes, and rapidly cooled in ice.

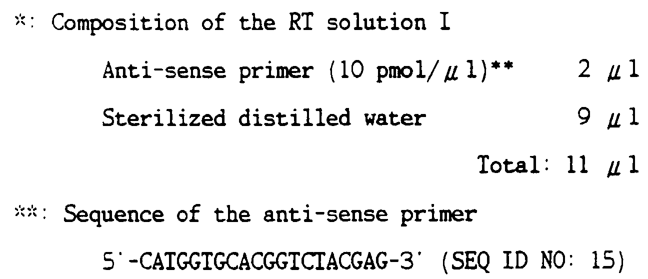

### (2) Reverse Transcription (Synthesis of cDNA)

The solution obtained in the above step (1) was briefly mixed by vortex, to which RT solution II* was added. This mixture was allowed to react at 37 °C for 30 minutes and then at 42 °C for 30 minutes, followed by brief centrifugation.

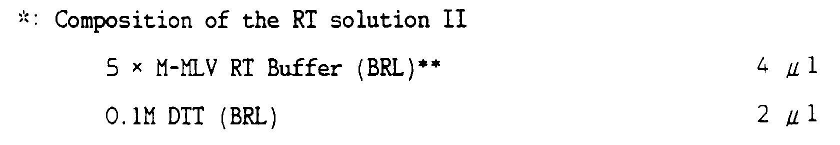

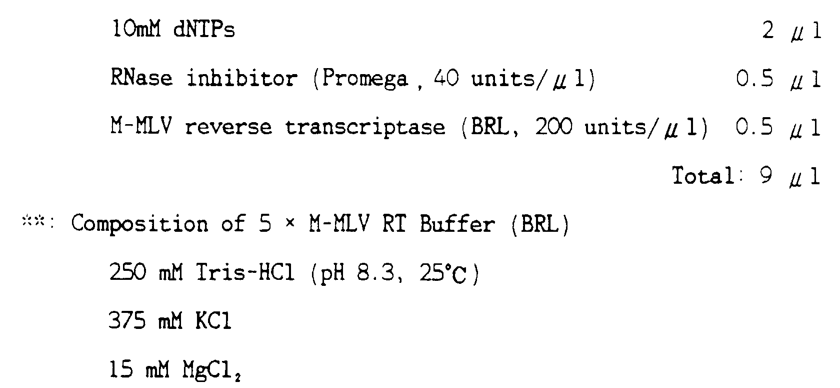

### (3) PCR

To 0.5 ml-eppendorf tube was added PCR solution*, to which was added 20 µl of cDNA solution of the above step (2) which had been heated at 95 °C for 5 minutes and rapidly cooled in ice.

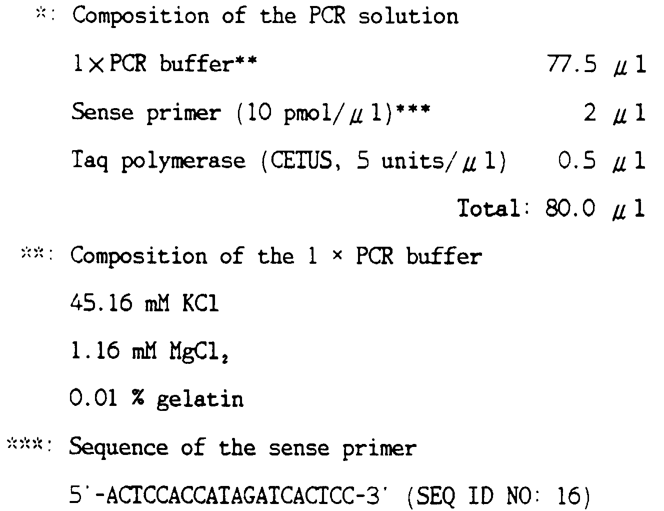

PCR was carried out 40 cycles with use of the solution obtained above and DNA Thermal Cycler (CETUS), one cycle being
Denaturation: 94 °C for 30 seconds,
Annealing: 55 °C for 1 minute, and
Extension: 72 °C for 1 minute,
followed by further extension at 72 °C for 10 minutes.

### (4) Southern Blotting

The PCR product obtained in the above (3) was electrophoresed by using 2 % agarose gel without ethidium bromide. The gel was immersed in 200 ml of 0.5 N NaOH - 1.5 M NaCl for 30 minutes with shaking slowly. DNA in the gel was alkali-blotted overnight to a membrane (Amersham, Hybond-N⁺) with use of 0.4 N NaOH as alkali trans buffer. The membrane was washed twice at room temperature for 15 minutes each with 2 × SSC with shaking slowly, further washed at 50 °C for 30 minutes, dried at room temperature, and then preserved at -20 °C.

### (5) Hybridization with ³²P-labeled HCV-cDNA

The membrane southern-blotted in the above (4) was immersed in a prehybridization solution (5 × SSPE, 5 × Denhardt's solution, 0.5 % SDS, and 100 µg/ml ssDNA) at 55 °C for 2 - 4 hours. A ³²P-labeled HCV-cDNA probe* was added thereto. This was incubated overnight at 55 °C. The membrane was washed with about 100 ml of 0.1 × SSC/0.1 % SDS solution at room temperature for 30 minutes with shaking slowly and then with about 100 ml of 0.1 × SSC/0.1 % SDS solution at 50 °C for 30 minutes with shaking slowly and subjected to autoradiography at -80 °C to detect HCV-RNA.
*: Sequence of the ³²P-labeled HCV-cDNA probe

5'-GAGTGTCGTGCAGCCTCCAGGTCC-3' (SEQ ID NO: 17)

### (6) Detection of Antibody C-100

Independently of the above experiment, detection of antibody C-100 was carried out on the same samples as used in the above experiment, using ELISA kit of Ortho Co.

### (7) Result

Antibody C-100-positive were 93 (81 %) of 115 cases of non-A, non-B type chronic liver disease. HCV-RNA was detected in 92 % (86/93) of antibody C-100-positive cases and 50 % (11/22) of antibody C-100-negative cases but not in any case of B type chronic liver disease.

Independently, the same experiment was carried out on 20 cases of C type chronic liver disease which was antibody C-100-positive. HCV-RNA-positive were 20 (100 %) of 20 cases.

Another experiment in the similar manner to the above was carried out on 20 samples.

From 100-500 µl each of sera obtained from patients with chronic hepatitis C (20 samples) which were positive for C-100 antibodies, RNA was extracted and precipitated with ethanol, followed by reverse transcription using an anti-sense primer of the sequence designated by SEQ ID NO: 2, resulting in a cDNA. Further, a sense primer of the sequence designated by SEQ ID NO: 3 was added and PCR was carried out 40 cycles. The PCR products were subjected to electrophoresis on a 2% agarose gel and blotted on a membrane, followed by hybridization analysis using ³²P-labeled DNA of the sequence designated by SEQ ID NO: 4 as a probe. The C-100 antibody was assayed with an ELISA kit available from Ortho Co.

As a result, 18 samples in the 20 samples exhibited HCV-RNA positive.

SEQUENCE LISTING
SEQ ID NO: 1
SEQUENCE LENGTH: 623 base pairs
SEQUENCE TYPE: nucleic acid
TOPOLOGY: linear
MOLECULE TYPE: Other nucleic acid, cDNA derived from virus RNA
ANTI-SENSE: No
ORIGINAL SOURCE
ORGANISM: hepatitis C virus

SEQUENCE DESCRIPTION
GGCTATACCG GCGACTTCGA CTCGGTGATC GACTGTAACA CATGTGTCAC TCAGACGGTC 60
GATTTCAGCT TGGATCCCAC CTTCACCATT GAGACGACGA CCGTGCCCCA AGATGCGGTG 120
TCGCGCACAC AGCGGCGAGG TAGGACTGGC AGAGGTAGGA GAGGCATCTA CAGGTTTGTG 180
ACTCCAGGGG AACGGCCCTC GGGCATGTTC GATTCCTCGG TCCTGTGTGA GTGTTATGAC 240
GCGGGCTGCG CTTGGTATGA GCTTACGCCC GCTGAGACCT CGGTTAGGTT GCGGGCTTAC 300
CTAAATACAC CAGGGTTGCC CGTCTGCCAG GACCATCTGG AGTTCTGGGA GAGCGTCTTC 360
ACAGGCCTCA CCCACATAGA TGCCCATTTC TTGTCCCAGA CCAAGCAGGC AGGAGACAAC 420
TTCCCCTACC TGGTAGCATA TCAAGCCACA GTGTGCGCCA GGGCTCAGGC TCCACCTCCA 480
TCGTGGGACC AAATGTGGAA GTATCTCACA CGGCTAAAAC CTACGCTGCA CGGGCCAACG 540
CCCCTGCTGT ATAGGCTAGG AGCCGTCCAA AATGAAGTCA TCCTCACACA CCCCATAACC 600
AAATACATCA TGACATGCAT GTC 623

SEQ ID NO: 2
SEQUENCE LENGTH: 20 base pairs
SEQUENCE TYPE: nucleic acid
TOPOLOGY: linear
MOLECULE TYPE: Other nucleic acid, synthesized DNA
ANTI-SENSE: Yes

SEQUENCE DESCRIPTION
GACATGCATG TCATGATGTA 20

SEQ ID NO: 3
SEQUENCE LENGTH: 20 base pairs
SEQUENCE TYPE: nucleic acid
TOPOLOGY: linear
MOLECULE TYPE: Other nucleic acid, synthesized DNA
ANTI-SENSE: No

SEQUENCE DESCRIPTION
GGCTATACCG GCGACTTCGA 20

SEQ ID NO: 4
SEQUENCE LENGTH: 293 base pairs
SEQUENCE TYPE: nucleic acid
TOPOLOGY: linear
MOLECULE TYPE: Other nucleic acid, synthesized DNA
ANTI-SENSE: No

SEQUENCE DESCRIPTION
GATCCCACCT TCACCATTGA GACGACGACC GTGCCCCAAG ATGCGGTGTC GCGCACACAG 60
CGGCGAGGTA GGACTGGCAG AGGTAGGAGA GGCATCTACA GGTTTGTGAC TCCAGGGGAA 120
CGGCCCTCGG GCATGTTCGA TTCCTCGGTC CTGTGTGAGT GTTATGACGC GGGCTGCGCT 180
TGGTATGAGC TTACGCCCGC TGAGACCTCG GTTAGGTTGC GGGCTTACCT AAATACACCA 240
GGGTTGCCCG TCTGCCAGGA CCATCTGGAG TTCTGGGAGA GCGTCTTCAC AGG 293

SEQ ID NO: 5
SEQUENCE LENGTH: 467 base pairs
SEQUENCE TYPE: nucleic acid

TOPOLOGY: linear
MOLECULE TYPE: Other nucleic acid, cDNA derived from virus RNA
ANTI-SENSE: No
ORIGINAL SOURCE
ORGANISM: hepatitis C virus

SEQUENCE DESCRIPTION
ACCAAACGTA ACACCAACCG TCGCCCACAG GACGTCAAGT TCCCGGGCGG TGGTCAGATC 60
GTTGGTGGAG TTTACCTGTT GCCGCGCAGG GGCCCCAGGT TGGGTGTGCG CGCGACTAGG 120
AAGACTTCCG AGCGGTCGCA ACCTCGTGGA AGGCGACAAC CTATCCCCAA GGCTCGCCGG 180
CCCGAGGGCA GGGCCTGGGC TCAGCCCGGG TACCCTTGGC CCCTCTATGG CAATGAGGGT 240
CTTGGGTGGG CAGGATGGCT CCTGTCACCC CGAGGCTCTC GGCCTAGTTG GGGCCCCACT 300
GACCCCCGGC GTAGGTCGCG TAATTTGGGT AAGGTCATCG ATACCCTCAC ATGCGGCTTC 360
GCCGACCTCA TGGGGTACAT TCCGCTCGTC GGCGCCCCCC TGGGAGGCGC TGCCAGGGCC 420
CTGGCGCATG GCGTCCGGGT TCTGGAGGAC GGCGTGAACT ATGCAAC 467

SEQ ID NO: 6
SEQUENCE LENGTH: 20 base pairs
SEQUENCE TYPE: nucleic acid
STRANDEDNESS: Single
TOPOLOGY: linear
MOLECULE TYPE: Other nucleic acid, synthesized DNA
ANTI-SENSE: Yes

SEQUENCE DESCRIPTION
AAGATAGAGA AAGAGCAACC 20

SEQ ID NO: 7
SEQUENCE LENGTH: 20 base pairs
SEQUENCE TYPE: nucleic acid

STRANDEDNESS: Single
TOPOLOGY: linear
MOLECULE TYPE: Other nucleic acid, synthesized DNA
ANTI-SENSE: No

SEQUENCE DESCRIPTION
CTCGTAGACC GTGCACCATG 20

SEQ ID NO: 8
SEQUENCE LENGTH: 20 base pairs
SEQUENCE TYPE: nucleic acid
STRANDEDNESS: Single
TOPOLOGY: linear
MOLECULE TYPE: Other nucleic acid, synthesized DNA
ANTI-SENSE: No

SEQUENCE DESCRIPTION
ACCAAACGTA ACACCAACCG 20

SEQ ID NO: 9
SEQUENCE LENGTH: 20 base pairs
SEQUENCE TYPE: nucleic acid
STRANDEDNESS: Single
TOPOLOGY: linear
MOLECULE TYPE: Other nucleic acid, synthesized DNA
ANTI-SENSE: Yes

SEQUENCE DESCRIPTION
GTTGCATAGT TCACGCCGTC 20

SEQ ID NO: 10

SEQUENCE LENGTH: 807 base pairs

SEQUENCE TYPE: nucleic acid

TOPOLOGY: linear

MOLECULE TYPE: Other nucleic acid, cDNA derived from virus RNA

ANTI-SENSE: No

ORIGINAL SOURCE

ORGANISM: hepatitis C virus

SEQUENCE DESCRIPTION

CCGGCGACAC TCCACCATAG ATCACTCCCC TGTGAGGAAC TACTGTCTTC ACGCAGAAAG 60

CGTCTAGCCA TGGCGTTAGT ATGAGTGTCG TGCAGCCTCC AGGTCCCCCC TCCCGGGAGA 120

GCCATAGAGG TCTGCGGAAC CGGTGAGTAC ACCGGAATTG CCAGGACGAC CGGGTCCTTT 180

CTTGGATCAA CCCGCTCAAT GCCTGGAGAT TTGGGCGTGC CCCCGCGAGA CTGCTAGCCG 240

AGTAGTGTTG GGTCGCGAAA GGCCTTGTGG TACTGCCTGA TAGGGTGCTT GCGAGTGCCC 300

CGGGAGGTCT CGTAGACCGT GCACCATGAG CACAAATCCT AAACCTCAAA GAAAAACCAA 360

ACGTAACACC AACCGCCGCC CACAGGACGT CAAGTTCCCG GGCGGTGGTC AGATCGTTGG 420

TGGAGTTTAC CTGTTGCCGC GCAGGGGCCC CAGGTTGGGT GTGCGCGCGA CTAGGAAGAC 480

TTCCGAGCGG TCGCAACCTC GTGGAAGGCG ACAACCTATC CCCAAGGCTC GCCGGCCCGA 540

GGGCAGGGCC TGGGCTCAGC CCGGGTACCC TTGGCCCCTC TATGGCAATG AGGGTCTTGG 600

GTGGGCAGGA TGGCTCCTGT CACCCCGAGG CTCTCGGCCT AGCTGGGGCC CCACTGACCC 660

CCGGCGTAGG TCGCGTAATT TGGGTAAGGT CATCGATACC CTCACATGCG GCTTCGCCGA 720

CCTCATGGGG TACATTCCGC TCGTCGGCGC CCCCCTGGGA GGCGCTGCCA GGGCCCTGGC 780

GCATGGCGTC CGGGTTCTGG AACACGG 807

SEQ ID NO: 11

SEQUENCE LENGTH: 20 base pairs

SEQUENCE TYPE: nucleic acid

STRANDEDNESS: Single

TOPOLOGY: linear

MOLECULE TYPE: Other nucleic acid, synthesized DNA
ANTI-SENSE: Yes

SEQUENCE DESCRIPTION
AAGATAGAGA AAGAGCAACC 20

SEQ ID NO: 12
SEQUENCE LENGTH: 20 base pairs
SEQUENCE TYPE: nucleic acid
STRANDEDNESS: Single
TOPOLOGY: linear
MOLECULE TYPE: Other nucleic acid, synthesized DNA
ANTI-SENSE: No

SEQUENCE DESCRIPTION
GGCGACACTC CACCATAGAT 20

SEQ ID NO: 13
SEQUENCE LENGTH: 20 base pairs
SEQUENCE TYPE: nucleic acid
STRANDEDNESS: Single
TOPOLOGY: linear
MOLECULE TYPE: Other nucleic acid, synthesized DNA
ANTI-SENSE: No

SEQUENCE DESCRIPTION
GGCGACACTC CACCATAGAT 20

SEQ ID NO: 14
SEQUENCE LENGTH: 20 base pairs

SEQUENCE TYPE: nucleic acid
STRANDEDNESS: Single
TOPOLOGY: linear
MOLECULE TYPE: Other nucleic acid, synthesized DNA
ANTI-SENSE: Yes

SEQUENCE DESCRIPTION
CCGTGTTCCA GAACCCGGAC 20

SEQ ID NO: 15
SEQUENCE LENGTH: 20 base pairs
SEQUENCE TYPE: nucleic acid
STRANDEDNESS: Single
TOPOLOGY: linear
MOLECULE TYPE: Other nucleic acid, synthesized DNA
ANTI-SENSE: Yes

SEQUENCE DESCRIPTION
CATGGTGCAC GGTCTACGAG 20

SEQ ID NO: 16
SEQUENCE LENGTH: 20 base pairs
SEQUENCE TYPE: nucleic acid
STRANDEDNESS: Single
TOPOLOGY: linear
MOLECULE TYPE: Other nucleic acid, synthesized DNA
ANTI-SENSE: No

SEQUENCE DESCRIPTION
ACTCCACCAT AGATCACTCC 20

SEQ ID NO: 17
SEQUENCE LENGTH: 24 base pairs
SEQUENCE TYPE: nucleic acid
STRANDEDNESS: Single
TOPOLOGY: linear
MOLECULE TYPE: Other nucleic acid, synthesized DNA

SEQUENCE DESCRIPTION
GAGTGTCGTG CAGCCTCCAG GTCC 24

## Claims (Claims for the following Contracting State(s): AT, BE, CH, DE, DK, FR, GB, GR, IT, LI, LU, NL, SE)

1. A DNA sequence comprising a DNA sequence designated by SEQ ID NO: 1, SEQ ID NO: 5, or SEQ ID NO: 10.

2. A DNA sequence according to claim 1, which is the DNA sequence designated by SEQ ID NO: 1, SEQ ID NO: 5, or SEQ ID NO: 10.

3. A method for the detection of hepatitis C virus, which is based on the PCR technique characterized by using a primer composed of from 15 to 40 bases including at least 15 straight bases in the DNA sequence designated by SEQ ID NO: 2 and a primer composed of from 15 to 40 bases including at least 15 straight bases in the DNA sequence designated by SEQ ID NO: 3; or using a primer composed of from 15 to 40 bases including at least 15 straight bases in the DNA sequence designated by SEQ ID NO: 15 and a primer composed of from 15 to 40 bases including at least 15 straight bases in the DNA sequence designated by SEQ ID NO: 16.

## Claims (Claims for the following Contracting State(s): ES)

1. A method for the preparation of a DNA sequence comprising a DNA sequence designated by SEQ ID NO: 1, SEQ ID NO: 5, or SEQ ID NO: 10, said method comprising the following steps:
(a) extraction of RNA from the sera of HCV-antibody positive patients and reverse transcription of said RNA to obtain a cDNA,
(b) amplification of said cDNA by PCR and cloning of the amplification products after gelelectrophoretic purification, and
(c) isolation of the DNA inserts of positive clones and determination of the sequence of said DNA.

2. The method according to claim 1, wherein said DNA sequence is the DNA sequence designated by SEQ ID NO: 1, SEQ ID NO: 5, or SEQ ID NO: 10.

3. A method for the detection of hepatitis C virus, which is based on the PCR technique characterized by using a primer composed of from 15 to 40 bases including at least 15 straight bases in the DNA sequence designated by SEQ ID NO: 2 and a primer composed of from 15 to 40 bases including at least 15 straight bases in the DNA sequence designated by SEQ ID NO 3; or using a primer composed of from 15 to 40 bases including at least 15 straight bases in the DNA sequence designated by SEQ ID NO: 15 and a primer composed of from 15 to 40 bases including at least 15 straight bases in the DNA sequence designated by SEQ ID NO: 16.

## Patentansprüche (Patentansprüche für folgende(n) Vertragsstaat(en): AT, BE, CH, DE, DK, FR, GB, GR, IT, LI, LU, NL, SE)

1. DNA-Sequenz, umfassend eine DNA-Sequenz mit der Bezeichnung SEQ ID NO: 1, SEQ ID NO: 5 oder SEQ ID NO: 10.

2. DNA-Sequenz nach Anspruch 1, die die DNA-Sequenz mit der Bezeichnung SEQ ID NO: 1, SEQ ID NO: 5 oder SEQ ID NO: 10 ist.

3. Verfahren zum Nachweis eines Hepatitis C-Virus, das auf der PCR-Technik beruht, dadurch gekennzeichnet, daß ein Primer verwendet wird, der aus 15 bis 40 Basen zusammengesetzt ist, einschließlich mindestens 15 ununterbrochenen Basen aus der DNA-Sequenz mit der Bezeichnung SEQ ID NO: 2, und ein Primer, der aus 15 bis 40 Basen zusammengesetzt ist, einschließlich mindestens 15 ununterbrochenen Basen aus der DNA-Sequenz mit der Bezeichnung SEQ ID NO: 3; oder ein Primer, der aus 15 bis 40 Basen zusammengesetzt ist, einschließlich mindestens 15 ununterbrochenen Basen aus der DNA-Sequenz mit der Bezeichnung SEQ ID NO: 15, und ein Primer, der aus 15 bis 40 Basen zusammengesetzt ist, einschließlich mindestens 15 ununterbrochenen Basen aus der DNA-Sequenz mit der Bezeichnung SEQ ID NO: 16.

## Patentansprüche (Patentansprüche für folgende(n) Vertragsstaat(en): ES)

1. Verfahren zur Herstellung einer DNA-Sequenz, umfassend eine DNA-Sequenz mit der Bezeichnung SEQ ID NO: 1, SEQ ID NO: 5 oder SEQ ID NO: 10, wobei das Verfahren die folgenden Schritte umfaßt:
(a) Extraktion von RNA aus Seren von HCV-Antikörperpositiven Patienten und reverse Transkription der RNA zum Erhalt einer cDNA,
(b) Amplifikation der cDNA durch PCR und Clonierung der amplifizierten Produkte nach gelelektrophoretischer Reinigung, und
(c) Isolierung der DNA Insertionen von positiven Clonen und Bestimmung der DNA-Sequenz.

2. Verfahren nach Anspruch 1, wobei die DNA-Sequenz die DNA-Sequenz mit der Bezeichnung SEQ ID NO: 1, SEQ ID NO: 5 oder SEQ ID NO: 10 ist.

3. Verfahren zum Nachweis eines Hepatitis C-Virus, das auf der PCR-Technik beruht, dadurch gekennzeichnet, daß ein Primer verwendet wird, der aus 15 bis 40 Basen zusammengesetzt ist, einschließlich mindestens 15 ununterbrochenen Basen aus der DNA-Sequenz mit der Bezeichnung SEQ ID NO: 2, und ein Primer, der aus 15 bis 40 Basen zusammengesetzt ist, einschließlich mindestens 15 ununterbrochenen Basen aus der DNA-Sequenz mit der Bezeichnung SEQ ID NO: 3; oder ein Primer, der aus 15 bis 40 Basen zusammengesetzt ist, einschließlich mindestens 15 ununterbrochenen Basen aus der DNA-Sequenz mit der Bezeichnung SEQ ID NO: 15, und ein Primer, der aus 15 bis 40 Basen zusammengesetzt ist, einschließlich mindestens 15 ununterbrochenen Basen aus der DNA-Sequenz mit der Bezeichnung SEQ ID NO: 16.

## Revendications (Revendications pour l'(les) Etat(s) contractant(s) suivant(s): AT, BE, CH, DE, DK, FR, GB, GR, IT, LI, LU, NL, SE)

1. Séquence d'ADN comprenant une séquence d'ADN désignée par SEQ ID NO: 1, SEQ ID NO: 5 ou SEQ ID NO: 10.

2. Séquence d'ADN selon la revendication 1, qui est la séquence d'ADN désignée par SEQ ID NO: 1, SEQ ID NO: 5 ou SEQ ID NO: 10.

3. Procédé de détection du virus de l'hépatite C, fondé sur la technique de la PCR, caractérisé en ce que l'on utilise une amorce composée de 15 à 40 bases comprenant au moins 15 bases alignées dans la séquence d'ADN désignée par SEQ ID NO: 2 et une amorce composée de 15 à 40 bases comprenant au moins 15 bases alignées dans la séquence d'ADN désignée par SEQ ID NO: 3, ou en ce que l'on utilise une amorce composée de 15 à 40 bases comprenant au moins 15 bases alignées dans la séquence d'ADN désignée par SEQ ID NO: 15 et une amorce composée de 15 à 40 bases comprenant au moins 15 bases alignées dans la séquence d'ADN désignée par SEQ ID NO: 16.

## Revendications (Revendications pour l'(les) Etat(s) contractant(s) suivant(s): ES)

1. Procédé de préparation d'une séquence d'ADN comprenant une séquence d'ADN désignée par SEQ ID NO: 1, SEQ ID NO: 5 ou SEQ ID NO: 10 ledit procédé comprenant les étapes suivantes:
(a) l'extraction d'ARN du sérum de patients positifs pour les anticorps dirigés contre le virus de l'hépatite C et la transcription inverse dudit ARN, ce qui donne un ADNc,
(b) l'amplification par PCR dudit ADNc et le clonage des produits de l'amplification après une purification par électrophorèse en gel, et
(c) l'isolement des inserts d'ADN de clones positifs et la détermination de la séquence desdits ADN.

2. Procédé selon la revendication 1, dans lequel ledit ADN est la séquence d'ADN désignée par SEQ ID NO: 1, SEQ ID NO: 5 ou SEQ ID NO: 10.

3. Procédé de détection du virus de l'hépatite C, fondé sur la technique de la PCR, caractérisé en ce que l'on utilise une amorce composée de 15 à 40 bases comprenant au moins 15 bases alignées dans la séquence d'ADN désignée par SEQ ID NO: 2 et une amorce composée de 15 à 40 bases comprenant au moins 15 bases alignées dans la séquence d'ADN désignée par SEQ ID NO: 3, ou en ce que l'on utilise une amorce composée de 15 à 40 bases comprenant au moins 15 bases alignées dans la séquence d'ADN désignée par SEQ ID NO: 15 et une amorce composée de 15 à 40 bases comprenant au moins 15 bases alignées dans la séquence d'ADN désignée par SEQ ID NO: 16.
